# EUROPÄISCHE PATENTANMELDUNG

(11) **EP 1 473 028 A1**
(43) Veröffentlichungstag der Anmeldung: **03.11.2004**
(21) Anmeldenummer: 04405098.7
(22) Anmeldetag: 23.02.2004
(51) Int. Cl.: A61K 7/48

(54) **Kosmetische Hautbehandlungsmittel und kosmetische Wirkstoffe zum Schutz gegen frühzeitige Hautalterung**

(30) Priorität: 30.04.2003 CH 7612003
(71) Anmelder: Mibelle AG, 5033 Buchs (CH)
(72) Erfinder: Schmid, Daniel, 5200 Brugg (CH); Schürch, Cornelia, 8105 Watt (CH); Zülli, Fred, Dr., 5024 Küttingen (CH)
(74) Vertreter: Rottmann, Maximilian R.

(57) **Zusammenfassung**

*Mycosporine-like amino acids* (MAAs), insbesondere solche aus der Rotalgenart *Porphyra umbilicalis*, werden in kosmetischen Hautbehandlungsmitteln zum Schutz gegen UVA-induzierte Lipidoxidation, welche zu frühzeitiger Hautalterung führt, eingesetzt.

## Beschreibung

Gegenstand der Erfindung sind:
- kosmetisches Hautbehandlungsmittel zum Schutz vor frühzeitiger Hautalterung wie sie in den Ansprüchen 1 bis 9 umschrieben sind;
- kosmetische Wirkstoffe zum Schutz vor frühzeitiger Hautalterung wie sie in den Ansprüchen 10 bis 17 umschrieben sind;
- gegen frühzeitige Hautalterung wirksame *Mycosporine-like amino acids* (MAAs).wie sie in den Ansprüchen 18 und 19 umschrieben sind; und
- die Verwendung von *Mycosporine-like amino acids* (MAAs) zur Herstellung von gegen vorzeitige Hautalterung wirksamen kosmetischen Hautbehandlungsmitteln wie sie in den Ansprüchen 20 und 21 umschrieben ist.

Beim Phänomen Hautalterung unterscheidet man zwischen chronologischem Altern (Intrinsic Aging) und dem durch die Umwelt beeinflusstem Altern (Extrinsic Aging). Intrinsic Aging beschreibt den natürlichen Alterungsprozess. Dafür werden verschiedene Faktoren verantwortlich gemacht, wie zum Beispiel Anhäufung von toxischen Sauerstoffradikalen, Abbau der Chromosomenenden (Telomere) oder kovalente Verbindungen von Zuckern mit Proteinen. Die natürliche Alterung führt zu einer Verdünnung aller Hautschichten, wobei die Haut dünn, trocken und feinrunzelig wird. Frühzeitige Hautalterung (Extrinsic Aging) ist vor allem eine Folge der Ultraviolett (UV)-Strahlung des Sonnenlichtes (Photoaging). Durch Licht gealterte Haut ist schlaff, stark runzelig und in grobe Falten gelegt.

Die UV-Strahlung der Sonne wird unterteilt in einen UVB-Bereich (280 bis 320 nm) und einen UVA-Bereich (320 bis 400 nm). Die UVB-Strahlen werden in der Hornschicht (Epidermis) absorbiert und dringen höchstens bis zur Basalmembran. Das UVB-Licht ist verantwortlich für Sonnenbrand (Sonnenerythem), Schädigung der Erbsubstanz (DNA) der Epidermiszellen und damit für die Entwicklung von Hautkrebs. Die längerwelligen, energieschwächeren UVA-Strahlen dringen tief in das Bindegewebe des Coriums (Dermis) ein. Dort führen sie über die Bildung von Sauerstoffradikalen und proinflammatorischen Cytokinen (IL-1β und TNF-α) zu einem Abbau der hauptsächlichen Strukturproteine des Bindegewebes, der Kollagene und der Elastine. Die Zerstörung dieser Proteine führt zu tiefen "Sonnenfalten". Die Bildung von Sauerstoffradikalen führt auch zur Peroxidation von Hautlipiden, insbesondere von Squalen. Die Lipide verlieren dadurch ihre Funktion (Y. Yamamoto, Journal of Dermatological Science 27Suppl: S1 bis S4, 2001). Das UVA-Licht ist somit hauptverantwortlich für die frühzeitige Alterung der Haut.

Die UVB-Strahlung ist morgens schwach, nimmt ab 11 Uhr zu, erreicht ihren Höhepunkt in der Mittagszeit und nimmt anschliessend rasch ab. Die Intensität der UVA-Strahlung entspricht eher derjenigen des sichtbaren Lichtes. UVA ist bereits beim Sonnenaufgang vorhanden und ebenso bei bedecktem Himmel. UVA kann nicht wie UVB durch Glas abgeblockt werden und ist deshalb auch drinnen, zu Hause oder am Arbeitsplatz, immer da. Die UVA-Strahlen stellen etwa 95 % der totalen UV-Strahlung, die Haut erreicht, dar. Es ist deshalb unerlässlich, einen alltäglichen Lichtschutz gegen UVA-Strahlen anzuwenden.

Moderne Tagescremen enthalten bereits UVA-Filter für den alltäglichen Schutz vor Hautalterung. Es sind allerdings bloss wenige, mit Mängel behaftete UVA-Filter erhältlich. Die konventionellen Butyl-methoxydibenzoyl-methan-Filter (z.B. Parsol® 1789) sind nicht lichtstabil. Der neue Bis-Ethylhexyloxyphenol-methoxyphenyltriazin-Filter (z.B. Tinosorb® S⁶) ist nicht sehr effizient. Beide Filter sind öllöslich und dringen daher sehr gut in tiefere Hautschichten ein. Dies ist nicht erwünscht, da sich die Filter und deren Abbauprodukte im Lörper anreichern können und Nebenwirkungen bewirken können. So wurden in Studien mit synthetischen UV-Filtern endokrine Nebeneffekte festgestellt (Schlumpf et al., Environmental Health Perspectives 109, 239 bis 244, 2001). In Sonnenschutzprodukten sind die Schutzeffekte der Filter bestimmt viel gewichtiger als deren mögliche Nebenwirkungen. Für den Einsatz in Tagescremen jedoch, die jahrelang täglich aufgetragen werden, sind nur absolut unbedenkliche Wirkstoffe geeignet.

Der vorliegenden Erfindung liegt die Aufgabe zu Grunde, ein kosmetisches Präparat zu schaffen, welches wirksam gegen UVA-induzierte Lipidperoxidation schützt und somit der Hautalterung entgegenwirkt, und welches völlig unbedenklich ist, so dass es jeden Tag angewendet werden kann. Diese Aufgabe wird durch die in den Ansprüchen 1 bis 19 umschriebenen Hautbehandlungsmittel, kosmetischen Wirkstoffe und Verwendungen gelöst.

Da UV-Strahlung die Erbsubstanz in den Zellen beschädigen kann, birgt sie für alle Lebewesen eine potentielle Gefahr. Bei vielen Tieren und auch beim Menschen übernehmen Melanine die Funktion der UV-Abschirmung. Melanine sind komplexe, aus Tyrosinen abgeleitete Moleküle, die neben UV-Licht auch sichtbares Licht absorbieren. Photosynthetisierende Organismen, wie Cyanobakterien (Blaualgen), Algen und höhere Pflanzen brauchen das sichtbare Licht zur Energiegewinnung und entwickelten zum UV-Schutz daher Moleküle, die spezifisch UV-Licht absorbieren. Bei den Pflanzen sind das Phenole, wie die Zimtsäurederivate und die Stoffe aus der Klasse der Flavonoide. In Cyanobakterien und Algen sind es die Substanzen Scytonemin und *Mycosporine-like amino acids* (MAAs), welche die Zellen vor Schädigung durch UV-Strahlung schützen (als Übersichtsartikel siehe: Ch. S. Cockell und J. Knowland, Biological Reviews of the Cambridge Philosophical Society, 1999, Band 74, Seiten 311 bis 345).

*Mycosporine-like amino acids* (MAAs) sind wasserlösliche Substanzen mit einem durchschnittlichen Molekulargewicht von 300 Gramm (Dalton). Sie sind charakterisiert durch einen Cyclohexanon- oder Cyclohexenimin-Chromophor, der mit Aminosäuren oder Iminoalkoholgruppen derivatisiert ist. MAAs absorbieren im Bereich von 310 bis 360 nm. MAAs wurden erstmals in Pilzsporen entdeckt. Unterdessen sind MAAs in Cyanobakterien, Rotalgen, Dinoflagellaten, Korallen und vielen marinen wirbellosen Tieren nachgewiesen. Die Absorptionskoeffizienten [mol⁻¹ I cm⁻¹] von MAAs sind sehr hoch und erreichen Werte von 40'000 (Porphyra-334 bei 334 nm) und 45'000 (Shinorine bei 334 nm) (siehe: A. Gröniger, R. P. Sinha, M. Klisch und D.-P. Häder, Journal of Photochemistry and Photobiology B: Biology, 2000, Band 58, Seiten 115-122). Diese natürlichen UV-Filter sind demnach ebenso effizient, wie die beiden synthetischen Filter Parsol® 1789 und Mexoryl® SX mit Peakabsorptionskoeffizienten von 40'000 und 45'000. Shinorine und Porphyra-334 zeichnen sich durch Photostabilität aus und dadurch, dass sie bei Anregung im Peakabsorptionsbereich nicht (Shinorine) oder nur sehr schwach fluoreszieren (Porphyra-334) (siehe: J. M. Shick und W. C. Dunlap, Annual Review of Physiology, 2002, Band 64, Seiten 223-262). Shinorine produzierte nach Anregung keine messbaren Radikalformen. Es sind also Substanzen, die UV-Energie nur in Form von Wärme wieder abgeben. Diese Eigenschaften zeigen, dass es sich bei MAAs um einzigartige, durch die Evolution hervorgebrachte, natürliche Sonnenfilter handelt.

Über den Einsatz von Mycosporinen als natürliche Sonnenschutzmittel gibt es bereits Veröffentlichungen. Die Veröffentlichungen WO 88/02251 und US 5 000 946 beschreiben die Anwendung von MAAs als Sonnenschutzmittel. Die Veröffentlichung WO 00/24369 schlägt vor, natürliche UV-absorbierende Substanzen aus der Gruppe der Carotinoide, Polyphenole und/oder MAAs als Sonnenschutzmittel anzuwenden. Die Veröffentlichung FR 2 803 200 schlägt vor, die MAAs der Rotalge *Polysiphonia lanosa* als topisches Lichtschutzmittel für Haut und Haare zu gebrauchen. Die Veröffentlichung WO 02/39974 beschreibt den Einsatz von MAAs als Sonnenschutzmittel in Pflegeprodukten. Die Anwendung von MAAs in Sonnenschutzprodukten ist demnach nicht neu. Beschrieben wurde auch, dass sie eine natürliche Alternative zu den synthetischen Filtern sind, vor allem bezüglich UVA, und dass MAAs als wasserlösliche, nicht-penetrierende Stoffe kein Gefahrenpotential aufweisen.

Unerwähnt allerdings blieb in all diesen Veröffentlichungen, dass *Mycosporine-like amino acids* (MAAs) kaum eine echte Alternative zu synthetischen Filter sein können, da die Gewinnung der MAAs viel zu aufwendig und kostspielig ist. Der Gehalt an MAAs in den Organismen ist sehr gering und erreicht normalerweise höchstens 1 % der Trockenmasse. Verglichen mit den synthetischen Filtern (durchschnittlicher Preis CHF 50,00/kg) wäre ein MAA-Präparat mit gleichem Absorptionsvermögen viel zu teuer. Die MAAs sind demnach nicht geeignet für die Anwendung in eigentlichen Sonnenschutzprodukten, die durch einen SPF-Faktor gekennzeichnet sind.

Es wurde nun gefunden, dass *Mycosporine-like amino acids* (MAAs) als Schutzmittel gegen frühzeitige Hautalterung für die Anwendung in täglichen Pflegeprodukten eingesetzt werden können. Die MAAs als natürliche, wasserlösliche, nicht-penetrierende Substanzen sind ungefährlich und können somit tagtäglich eingesetzt werden. Eigene Untersuchungen haben erstaunlicherweise gezeigt, dass für den Schutz vor Photoaging im Alltagsleben, zum Beispiel während eines normalen Arbeitstages, die Anwendung eines Produktes mit 0.005 % MAAs genügt.

Weiter wurde gefunden, dass mit der Rotalgenart *Porphyra umbilicalis* ein Material zur Verfügung steht, das wegen seines aussergewöhnlich hohen Gehaltes an *Mycospo* *rine-like amino acids* (MAAs), erstmals eine wirtschaftlich interessante Gewinnung von MAAs erlaubt.

Die erfindungsgemässen kosmetischen Hautbehandlungsmittel zum Schutz gegen UVA-indizierte Lipidoxidation, welche zu frühzeitiger Hautalterung führt, sind gekennzeichnet durch einen Gehalt an *Mycosporine-like amino acids* (MAAs). Vorzugsweise beträgt der Gehalt an *Mycosporine-like amino acids* (MAAs) 0,00001 bis 0,5 Gewichtsprozent. Die erfindungsgemässen Hautbehandlungsmittel können ausserdem mindestens ein Antioxidans, welches die Lipidoxidation durch einen anderen Mechanismus hemmt, vorzugsweise Substanzen aus der Gruppe der Zimtsäurederivate, der Polyphenole und der Carotinoide, sowie Natium-carboxymethyl-β-glucan, welches die Lipidoxidation durch einen weiteren Mechanismus hemmt, enthalten. Sodann können sie ausserdem Genistein enthalten, welches den UV-induzierten Kollagenabbau hemmt.

Die in den erfindungsgemässen Hautbehandlungsmitteln enthaltenen *Mycosporine-like amino acids* (MAAs) stammen vorzugsweise aus der Rotalgenart *Porphyra umbilicalis.* Vorzugsweise enthalten die erwähnten Hautbehandlungsmittel die genannten Wirkstoffe oder einzelne von ihnen in Liposomen eingebaut, damit sie zur Ereichung einer optimalen Funktion tiefer in das *Stratum corneum* hineingebracht werden können. Die genannten Hautbehandlungsmittel können ausserdem UVB- und UVA-Filter und/oder Pigmente, wie Titaniumdioxid und/oder Zinkoxid, enthalten.

Rotalgen *(Rhodophyceae)* bilden eine Klasse mit ca. 4000 Arten, die fast ausschliesslich im Meer vorkommen und dort vor allem die Litoralzonen besiedeln. Die Art *Porphyra umbilicalis,* bekannt unter den Namen Laver, Purple Laver oder Nori, wird seit jeher vom Menschen als "Seavegetable" genutzt, da sie eine reiche Quelle für Vitamine und Mineralien ist. Die Alge wird ca. 20 cm lang, ist zu Beginn grün, wird dann purpurfarbig und schliesslich schwarz. Als Organismen, die im Bereich der Gezeiten wachsen, sind *Porphyra*-Arten extrem der Sonnenstrahlung ausgesetzt. Es ist daher nicht verwunderlich, dass man in vielen *Porphyra*-Arten die MAAs *Porphyra*-334 und Shinorine nachweisen konnte (siehe: A. Gröniger, R. P. Sinha, M. Klisch und D.-P. Häder, Journal of Photochemistry and Photobiology B: Biology, 2000, Band 58, Seiten 115-122).

In *Porphyra umbilicalis* wurde ein MAA-Gehalt von 1 bis 2 % der Trockenmasse gefunden. Der MAA-Gehalt ist in diesem Fall als sehr hoch anzusehen, da man in anderen Organismen nur Werte zwischen 0,2 und 0,8 % gefunden hatte (siehe: Ch. S. Cockell und J. Knowland, Biological Reviews of the Cambridge Philosophical Society, 1999, Band 74, Seiten 311 bis 345). Die MAAs lassen sich durch Extraktion von getrocknetem *Porphyra*-Material mit Wasser, Ethanol, Butylenglykol oder einem Gemisch aus diesen Extraktionsmitteln gewinnen. Vorzugsweise wird das *Porphyra*-Material in einer Menge von 1 bis 10 % im Extraktionsmittel aufgenommen und während 1 bis 10 Stunden bei 20 bis 50 °C unter Rühren inkubiert. Für eine hohe Ausbeute wird vor der Extraktion vorzugsweise zuerst ein Zellaufschluss durchgeführt, indem das Material z.B. enzymatisch, z.B. mit Zellulasen oder Pektinasen, behandelt wird, oder indem das Material in flüssigem CO₂ gefroren und dann zerrieben wird, oder indem das Material mit Ultraschall behandelt wird. Danach wird durch Filtration oder Extraktion das feste Material vom Extrakt abgetrennt. Der Extrakt wird danach geklärt, vorzugsweise enzymatisch mittels Proteinasen oder Pektinasen, oder durch Ultrafiltration durch eine Membran mit einer Ausschlussgrösse von 10'000 Gramm (Dalton) bis 0,16 µm. Der geklärte Extrakt kann abschliessend durch Sprüh- oder Gefriertrocknung getrocknet werden.

Die Wirksamkeit von MAAs von *Porphyra umbilicalis* als Schutz gegen UVA-induzierte Lipidperoxidation, die zu frühzeitiger Hautalterung führt, konnte in einer Untersuchung am Menschen gezeigt werden. Die Untersuchung wurde mit 20 Frauen im Alter zwischen 36 und 54 durchgeführt. Die Testprodukte mussten während 4 Wochen täglich zweimal auf definierten Feldern angewendet werden, zur Messung von Rauhigkeit, Elastizität, Feuchtigkeit und Bildung von Squalenperoxiden auf der Innenseite des Vorderarmes und zur Messung von Faltentiefe auf dem Gesicht. Ein unbehandeltes Feld diente als Kontrolle. Alle Testfelder wurden zweimal wöchentlich mit UVA bestrahlt (10 J/cm²).

Eine Testcrème mit 5 % eines MAA-Präparates, das 0,1 % Porphyra und Shinorine (im Verhältnis 92:8) in Liposomen (3 % Lecithin) enthielt wurde gegen eine Creme mit synthetischen Filtern (4 % Neo Heliopan AV, 1 % Parsol 1789) und gegen die Crème allein verglichen. Die Anwendung der MAA-Crème führte trotz der UVA-Bestrahlung zu einer Verbesserung der Hautfestigkeit von 10 % (Crème mit synthetischen Filtern 7 %), der Hautrauhigkeit von 12 % (Crème mit synthetischen Filtern 6 %) und der Hautfeuchtigkeit von 15 % (Crème mit synthetischen Filtern 9 %). Die Faltentiefe war bei Anwendung der MAA-Crème nach 4 Wochen 18 % reduziert. Die UVA-induzierte Lipidperoxidation, gemessen als Squalenperoxide, konnte durch die MAA-Crème um 37 % reduziert werden (Crème mit synthetischen Filtern 35 %).

Alle im Folgenden genannten Zahlen sind Gewichts-Prozent. Die Bezeichnung der Inhaltsstoffe entspricht hauptsächlich der INCI- Nomenklatur (International Cosmetics Ingredients), wie sie zur Kennzeichnung kosmetischer Wirk- und Hilfsstoffe bestimmt ist.

### Beispiele

| | **Tagescreme** |
|---|---|
| Ethylhexyl Cocoate | 5,0 % |
| Cetyl Alcohol | 2,5 % |
| PEG-20 Methyl Glucose Sesquistearate | 2,5 % |
| Stearyl Alcohol | 2,5 % |
| Coco-Caprylate/Caprate | 2,0 % |
| Isohexadecane | 2,0 % |
| Palmitic / Stearic Acid | 2,0 % |
| Glycerin | 2,0 % |
| Methyl Glucose Sesquistearate | 1,7 % |
| Mycosporine-like amino acids (MAAs) | 0,005 % |
| Aqua, Parfüm und Konservierungsmittel | ad 100 % |

| | **Feuchtigkeitscreme** |
|---|---|
| Caprylic / Capric Triglyceride | 5,0 % |
| Vitis Vinifera (Grape) Seed Extract | 5,0 % |
| Glyceryl Stearate | 4,0 % |
| Paraffinum Liquidum | 2,0 % |
| Glycerin | 2,0 % |
| Stearyl Alcohol | 1,5 % |
| Cetyl Alcohol | 1,5 % |
| Palmitic / Stearic Acid | 1,0 % |
| Hydrogenated Polyisobutene | 0,5 % |
| Carbomer | 0,15 % |
| Sodium Hydroxide | 0,12 % |
| Glucosylrutin | 0,1 % |
| Disodium EDTA | 0,1 % |
| Sodium Carboxymethyl Beta-Glucan | 0,001 % |
| Mycosporine-like amino acids (MAAs) | 0,0005 % (in Liposomen) |
| Aqua, Parfüm und Konservierungsmittel | ad 100 % |

| | **Augenfaltengel** |
|---|---|
| Glycerin | 5,0 % |
| Pentylene Glycol | 5,0 % |
| Sorbitol | 5,0 % |
| Panthenol | 1,0 % |
| Xanthan Gum | 1,0 % |
| Carbomer | 0,2 % |
| Sodium Hydroxide | 0,2 % |
| Tocopherol | 0,1 % |
| Mycosporine-like amino acids (MAAs) | 0,05 % |
| Ubiquinone | 0,01 % |
| Aqua, Parfüm und Konservierungsmittel | ad 100 % |

| | **Haar Styling Gel** |
|---|---|
| Glycerin | 5,0 % |
| PVPNA Copolymer | 4,0 % |
| Alcohol | 2,0 % |
| Carbomer | 1,0 % |
| PEG-40 Hydrogenated Castor Oil | 1,0 % |
| Vitis Vinifera (Grape) Seed Extract | 0,5 % |
| Sodium Hydroxide | 0,2 % |
| Tocopherol | 0,01 % |
| Mycosporine-like amino acids (MAAs) | 0,001 % |
| Aqua, Parfüm und Konservierungsmittel | ad 100 % |

| | **Sonnencreme** |
|---|---|
| Mineral Oil | 9,0 % |
| PPG-15 Stearyl Ether | 6,0 % |
| Titanium Dioxide | 5,0 % |
| Zinc Oxide | 5,0 % |
| Octyl Salicylate | 3,0 % |
| Jojoba Oil | 3,0 % |
| Cocoa Butter | 2,0 % |
| Hydrogenated Castor Oil | 1,4 % |
| Sorbitane Stearate | 1,2 % |
| Mycosporine-like amino acids (MAAs) | 0,5 % (in Liposomen) |
| Soy Isoflavones | 0,005 % (in Liposomen) |
| Tocopherol | 0,001 % (in Liposomen) |
| Aqua, Parfüm und Konservierungsmittel | ad 100 % |

| | **Sonnencreme** |
|---|---|
| Mineral Oil | 9,0 % |
| Ethylhexyl Methoxycinnamate | 8,0 % |
| PPG-15 Stearyl Ether | 6,0 % |
| Octyl Salicylate | 3,0 % |
| Jojoba Oil | 3,0 % |
| Cocoa Butter | 2,0 % |
| Butyl Methoxydibenzoylmethane | 1,5 % |
| Hydrogenated Castor Oil | 1,4 % |
| Sorbitane Stearate | 1,2 % |
| Mycosporine-like amino acids (MAAs) | 0,1 % |
| Sodium Carboxymethyl Beta-Glucan | 0,1 % |
| Aqua, Parfüm und Konservierungsmittel | ad 100 % |

## Patentansprüche

1. Kosmetisches Hautbehandlungsmittel zum Schutz gegen UVA-induzierte Lipidoxidation, welche zu frühzeitiger Hautalterung führt, **gekennzeichnet durch** einen Gehalt an *Mycosporine-like amino acids* (MAAs).

2. Hautbehandlungsmittel nach Anspruch 1, **dadurch gekennzeichnet, dass** es 0,00001 bis 0,5 Gewichtsprozent *Mycosporine-like amino acids* (MAAs) enthält.

3. Hautbehandlungsmittel nach Anspruch 1 oder 2, **dadurch gekennzeichnet, dass** es ausserdem mindestens ein Antioxidans enthält, welches die Lipidoxidation durch einen anderen Mechanismus hemmt.

4. Hautbehandlungsmittel nach einem der Ansprüche 1 bis 3, **dadurch gekennzeichnet, dass** es ausserdem Natrium-carboxymethyl-β-glucan enthält.

5. Hautbehandlungsmittel nach einem der Ansprüche 1 bis 4, **dadurch gekennzeichnet, dass** es ausserdem Genistein enthält.

6. Hautbehandlungsmittel nach einem der Ansprüche 1 bis 5, **dadurch gekennzeichnet, dass** es *Mycosporine-like amino acids* (MAAs) aus der Rotalgenart *Porphyra umbilicalis* enthält.

7. Hautbehandlungsmittel nach einem der Ansprüche 1 bis 6, **dadurch gekennzeichnet, dass** es die genannten Wirkstoffe oder einzelne von ihnen in Liposomen eingebaut enthält.

8. Hautbehandlungsmittel nach einem der Ansprüche 1 bis 7, **dadurch gekennzeichnet, dass** es ausserdem UVB- und UVA-Filter und/oder Pigmente enthält,

9. Hautbehandlungsmittel nach Anspruch 8, **dadurch gekennzeichnet, dass** es als Pigmente Titaniumdioxid und/oder Zinkoxid enthält.

10. Kosmetischer Wirkstoff gegen UVA-induzierte Lipidoxidation, welche zu frühzeitiger Hautalterung führt, **gekennzeichnet durch** einen Gehalt an *Mycosporine-like amino acids* (MAAs).

11. Kosmetischer Wirkstoff nach Anspruch 10, **dadurch gekennzeichnet, dass** er ausserdem ein Antioxidans enthält.

12. Kosmetischer Wirkstoff nach Anspruch 10 oder 11, **dadurch gekennzeichnet, dass** er ausserdem Genistein enthält.

13. Kosmetischer Wirkstoff nach einem der Ansprüche 10 bis 12, **dadurch gekennzeichnet, dass** er ausserdem Natrium-carboxymethyl-β-glucan enthält.

14. Kosmetischer Wirkstoff nach einem der Ansprüche 10 bis 13, **dadurch gekennzeichnet, dass** er *Mycosporine-like amino acids* (MAAs) aus der Rotalgenart *Porphyra umbilicalis* enthält.

15. Kosmetischer Wirkstoff nach einem der Ansprüche 10 bis 14, **dadurch gekennzeichnet, dass** er die genannten Wirkstoffe oder einzelne von ihnen in Liposomen eingebaut enthält.

16. Kosmetischer Wirkstoff nach einem der Ansprüche 10 bis 15, **dadurch gekennzeichnet, dass** er ausserdem UVB- und UVA-Filter und/oder Pigmente enthält,

17. Kosmetischer Wirkstoff nach Anspruch 16, **dadurch gekennzeichnet, dass** er als Pigmente Titaniumdioxid und/oder Zinkoxid enthält.

18. *Mycosporine-like amino acids* (MAAs) als gegen vorzeitige Hautalterung wirksame kosmetische Wirkstoffe.

19. *Mycosporine-like amino acids* (MAAs) aus der Rotalgenart *Porphyra umbilicalis* als kosmetische Wirkstoffe nach Anspruch 16.

20. Verwendung von *Mycosporine-like amino acids* (MAAs) zur Herstellung von gegen vorzeitige Hautalterung wirksamen kosmetischen Hautbehandlungsmitteln,

21. Verwendung von *Mycosporine-like amino acids* (MAAs) aus der Rotalgenart *Porphyra umbilicalis* nach Anspruch 20.
